# FASCICULE DE BREVET EUROPEEN

(11) **EP 1 169 032 B1**
(45) Date de publication et mention de la délivrance du brevet: **31.08.2005**
(21) Numéro de dépôt: 00922718.2
(22) Date de dépôt: 21.04.2000
(51) Int. Cl.: A61K 31/192, A61K 9/58, A61P 29/00

(54) **MICROGRANULES DE KETOPROFENE, LEUR PROCEDE DE PREPARATION ET COMPOSITIONS PHARMACEUTIQUES**
KETOPROFEN MIKROGRANULAT, VERFAHREN ZUR HERSTELLUNG DESSELBEN SOWIE PHARMAZEUTISCHE ZUSAMMENSETZUNGEN
KETOPROFEN MICROGRANULES, METHOD FOR PREPARING THE SAME AND PHARMACEUTICAL COMPOSITIONS

(30) Priorité: 22.04.1999 FR 9905103
(43) Date de publication de la demande: 09.01.2002
(73) Titulaire: ETHYPHARM, 78550 Houdan (FR)
(72) Inventeur: LEBON, Christophe, F-28260 Rouvres (FR); MARECHAL, Dominique, F-28100 Dreux (FR); SUPLIE, Pascal, F-27400 Montaure (FR)
(74) Mandataire: Ahner, Francis
(86) Numéro de dépôt international: PCT/FR2000/001066
(87) Numéro de publication internationale: WO 2000/064432

(56) Documents cités:
- US-A- 4 980 170
- M. DITTGEN, A. BAUER, C. TIMPE AND H. OSTERWALD: "Influence of the pellet coating on the drug release from ketoprofen slow release capsule" PROCEEDINGS WORLD MEETING ON PHARMACEUTICS, BIOPHARMACEUTICS AND PHARMACEUTICAL TECHNOLOGY, vol. 1, 1995, pages 385-6, XP000874017
- HUSSON I ET AL: "MODELLING OF DRUG RELEASE FROM PELLETS COATED WITH AN INSOLUBLE POLYMERIC MEMBRANE" JOURNAL OF CONTROLLED RELEASE,NL,ELSEVIER SCIENCE PUBLISHERS B.V. AMSTERDAM, vol. 17, no. 2, page 163-173 XP000232472 ISSN: 0168-3659

## Description

La présente invention concerne une nouvelle formulation galénique du kétoprofène à libération prolongée pour administration orale journalière sous forme de microgranules.

Elle s'étend en outre à leur procédé de préparation et aux compositions pharmaceutiques les contenant.

Le kétoprofène, ou acide 2-(3-benzoyl-phényl)-propionique, est connu depuis 1972 comme médicament anti-inflammatoire non-stéroïdien. Il est couramment utilisé pour ses propriétés analgésiques, antipyrétiques et anti-inflammatoires.

Le kétoprofène est généralement administré pour le traitement symptomatique à long terme des maladies rhumatismales inflammatoires chroniques et de certaines ostéoarthrites, ainsi que pour le traitement symptomatique à court terme des déclenchements aigus de rhumatismes abarticulaires, des arthrites micro-crystalline, des ostéoarthrites, des lumbagos et des radiculalgies sévères.

La posologie usuelle varie de 50 à 300 mg, de préférence 100 à 200 mg par jour en une ou plusieurs doses.

Etant donné que le kétoprofène est fréquemment utilisé dans les traitements à long terme, et que sa demi-vie est courte (environ 2 heures), l'utilité de formes à libération prolongée s'impose.

Le document EP-A-403 383 décrit des granules à libération prolongée qui comprennent chacun un coeur comprenant du kétoprofène et de la cellulose microcristalline et un enrobage comprenant un dérivé cellulosique hydrosoluble et un dérivé cellulosique non hydrosoluble. La proportion massique de kétoprofène est comprise entre 60 à 80 %, elle est de préférence égale à 75 %. Les coeurs de granules sont obtenus par extrusion-sphéronisation et leur enrobage est réalisé en lit d'air fluidisé en utilisant une solution des deux dérivés cellulosiques dans le mélange MeOH/CH₂Cl₂.

Les granules décrits dans EP-A-403 383, par exemple la spécialité Profénid® LP 200 mg, présentent le désavantage d'être obtenu par un procédé qui utilise des solvants organiques.

Le document EP-A-361 910 décrit un des granulés dispersibles préparés par adsorption d'un principe actif pulvérulant de granulométrie inférieure à 100 microns, sur un excipient pulvérulent de granulométrie comprise entre 250 et 500 microns. Le principe actif peut être le kétoprofène et l'excipient peut être le lactose. Les particules obtenues sont mélangées à un excipient solide à température ambiante qui présente un point de fusion bas, par exemple l'acide stéarique ou un dérivé du polyéthylène glycol. Le mélange est chauffé jusqu'à ce que cet excipient fonde, et refroidi pour resolidifier ce même excipient une fois fixé sur les particules de principe actif.

Les granulés décrits dans EP-A-361 910 contiennent des teneurs en principe actif de l'ordre de 25 %, et apparaissent limités à ces faibles teneurs.

Le document EP-A-204 596 décrit des granules de kétoprofène à libération prolongée obtenus par extrusion d'un mélange de kétoprofène, d'un ou plusieurs polymères érodibles, d'au moins deux excipients lipidiques dont l'un possède la propriété de gélifier le ou les polymères et l'autre possède des propriétés lubrifiantes. Le polymère est par exemple l'éthylcellulose, un acrylate ou un copolymère vinylpyrrolidone/acétate de vinyle. Les excipients lipidiques sont des acides gras ou des huiles végétales.

Les granules décrits dans EP-A-204 596 présentent désavantageusement des profils de libération extrêmement variables d'une formulation à l'autre.

Le document EP-A-667 148 décrit des comprimés de kétoprofène à libération retardée constitués d'un coeur de principe actif enrobé d'un polymère cationique puis d'un polymère anionique. Le polymère cationique est soluble à un pH inférieur ou égal à 6, par exemple un copolymère méthacrylate d'aminoalkyle. Le polymère anionique est soluble à un pH supérieur ou égal à 5,5. Le polymère anionique peut être choisi parmi un copolymère acide méthacrylique/méthacrylate L (Eudragit L®), un copolymère acide méthacrylique/méthacrylate S (Eudragit S®), l'hydroxypropylméthylcellulose.

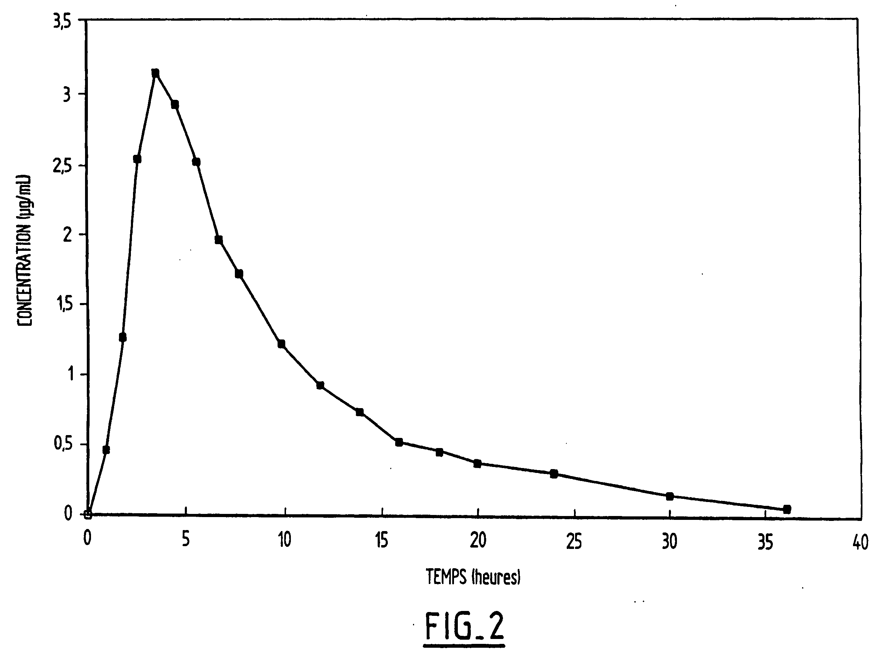

L'étude de biodisponibilité de cette formulation (figure 2 du document EP-A-667 148) montre que la concentration plasmatique en kétoprofène est quasiment nulle pendant les 13 heures suivant l'administration du comprimé contenant 50 mg de kétoprofène puis augmente linéairement jusqu'à 1,5 µg/ml pendant les 3 heures suivantes.

La spécialité ORUVAIL® 200 mg est fabriquée à partir de granulés composés de saccharose et d'amidon, enrobés de shellac et d'éthylcellulose. Les produits contenant du shellac posent cependant des problèmes de stabilité. En outre, la préparation d'une formulation contenant du shellac et de l'éthylcellulose nécessite l'utilisation de solvant organique.

L'objet de la présente invention est de fournir une formulation orale de kétoprofène à libération prolongée dépourvue des inconvénients de l'art antérieur, i.e.
- dont la préparation ne met en oeuvre aucun solvant organique, et
- dont les excipients sont chimiquement compatibles pour conférer une bonne stabilité dans le temps à la formulation.

C'est pourquoi, la présente invention porte sur des microgranules de kétoprofène à libération prolongée, chacun constitués d'un noyau actif contenant du kétoprofène enrobé d'une couche permettant la libération prolongée du principe actif caractérisés en ce que la couche entérique contient de l'Eudragit RL-30D® et de l'Eudragit RS-30D® dans une proportion massique comprise entre 80/20 et 95/5, de préférence, sensiblement égale à 90/10.

L'Eudragit RL-30D® et l'Eudragit RS-30D® sont des dispersions aqueuses à 30 % du copolymère poly (éthyl acrylate, méthyl méthacrylate, chlorure de triméthylammonio-éthyl méthacrylate) respectivement dans les proportions 1/2/0,2 et 1/2/0,1.

L'association de l'Eudragit RL-30D® et de l'Eudragit RS-30D® selon l'invention présente de nombreux avantages.

Eudragit RL-30D® et Eudragit RS-30D® sont miscibles en toute proportion, dans l'eau et ne nécessitent pas l'utilisation de solvants organiques.

Le film obtenu à partir de Eudragit RL-30D® et Eudragit RS-30D® est insoluble dans l'eau et dans les fluides digestifs, si bien que le principe actif est libéré par diffusion ; en outre, la perméabilité de ce film est indépendante du pH, de sorte que la libération du principe actif est indépendante des variations locales et individuelles du tube digestif.

Les propriétés du film contenant Eudragit RL-30D® et Eudragit RS-30D® sont parfaitement adaptées à l'enrobage de granules.

La masse totale d'Eudragit RL-30D® et d'Eudragit RS-30D® contenus dans la couche polymérique représente entre 1 à 8 % de la masse du microgranule, de préférence 2 et 5 %.

De façon avantageuse, la couche polymérique enrobant le noyau contient un agent plastifiant, comme le triéthylcitrate, et/ou un agent lubrifiant, choisi parmi les lubrifiants pharmaceutiquement acceptables, comme la silice anhydre colloïdale ou Syloid®.

Le plastifiant améliore l'élasticité du film polymérique et diminue la température à laquelle le film se forme. Le plastifiant représente 10 à 20 % en poids de la masse sèche des Eudragit RL-30D® et RS-30D®.

L'agent lubrifiant représente 10 à 30 % en poids de la masse sèche des Eudragit RL-30D® et RS-30D®.

La masse de kétoprofène contenu dans le noyau actif représente 50 à 80 % de la masse du microgranule, de préférence 65 à 75 %, de préférence encore 70 % environ.

Le noyau actif est avantageusement constitué d'un grain support neutre enrobé de kétoprofène et d'un agent liant, choisi parmi les liants pharmaceutiquement acceptables, par exemple un polyacrylate. Dans ce mode de réalisation, le grain support neutre représente 10 à 40 % en poids de la masse du microgranule, de préférence 20 à 25 %.

Le noyau actif des microgranules de l'invention contient de préférence de l'Eudragit® NE 30 D comme liant.

L'Eudragit® NE est une dispersion aqueuse à 30 % d'un copolymère acrylique : poly (éthyl acrylate, méthyl méthacrylate) dans les proportions 2/1.

L'Eudragit® NE présente l'avantage d'être chimiquement compatible avec les Eudragit RL-30D® et RS-30D® contenus dans la couche entérique. Il forme un film insoluble dans l'eau et dans les fluides digestifs.

La présente invention a également pour objet un procédé de préparation des microgranules de kétoprofène à libération prolongée présentés plus haut.

Le procédé selon l'invention est caractérisé en ce qu'il comprend une étape d'enrobage du noyau actif par pulvérisation d'une suspension aqueuse contenant de l'Eudragit RS-30D® et de l'Eudragit RL-30D®.

Le noyau actif est avantageusement obtenu par montage du kétoprofène sur des grains supports neutres avec une suspension aqueuse d'un agent liant comme un polyacrylate.

Selon un mode de réalisation préféré, une première étape du procédé consiste à préparer une suspension dite de montage constituée d'une suspension aqueuse d'Eudragit® NE 30D®, à 30 % en poids.

Le montage du kétoprofène est effectué dans une turbine à dragéifcation sur des grains supports neutres, par pulvérisation discontinue de la suspension décrite ci-dessus en alternance avec des séquences de poudrage du kétoprofène.

Les microgranules sont ensuite tamisés et séchés à 60°C environ pendant au moins 10 heures.

Une deuxième étape consiste à préparer une suspension d'enrobage constituée d'une solution aqueuse à 50 % en poids d'un mélange d'Eudragit RL-30D®, d'Eudragit RS-30D®, de silice et de triéthylcitrate dans des proportions massiques comprises entre 8/2/2/2 et 9,5/0,5/2/2, de préférence égales à 9/1/2/2.

Les microgranules à enrober sont ensuite placés dans une turbine à dragéification et pulvérisés de façon continue avec la suspension d'enrobage décrite précédemment.

Les microgranules enrobés sont ensuite tamisés et séchés à environ 40°C pendant 4 à 8 heures.

L'enrobage peut être effectué en plusieurs étapes selon la cinétique désirée.

Les microgranules séchés peuvent éventuellement être lubrifiés avec du talc, puis placés dans des gélules ou comprimés, contenant 50 à 300 mg, de préférence 100 mg à 200 mg de kétoprofène.

La présente invention concerne également toute composition pharmaceutique qui contient les microgranules décrits précédemment, susceptibles d'être obtenus par le procédé qui vient d'être présenté, la masse des microgranules contenus dans ladite composition correspondant à une dose journalière de 50 à 300 mg, de préférence 100 à 200 mg environ de kétoprofène.

Le profil de dissolution in vivo des microgranules et des gélules selon l'invention est tel que
- 15 à 35 % du principe actif, de préférence 20 à 30 %, sont libérés au bout de deux heures,
- 45 à 70 % du principe actif, de préférence 50 à 65 %, sont libérés au bout de 6 heures,
- plus de 65 % du principe actif, de préférence 70 %, voire même 80 %, sont libérés au bout de 12 heures.

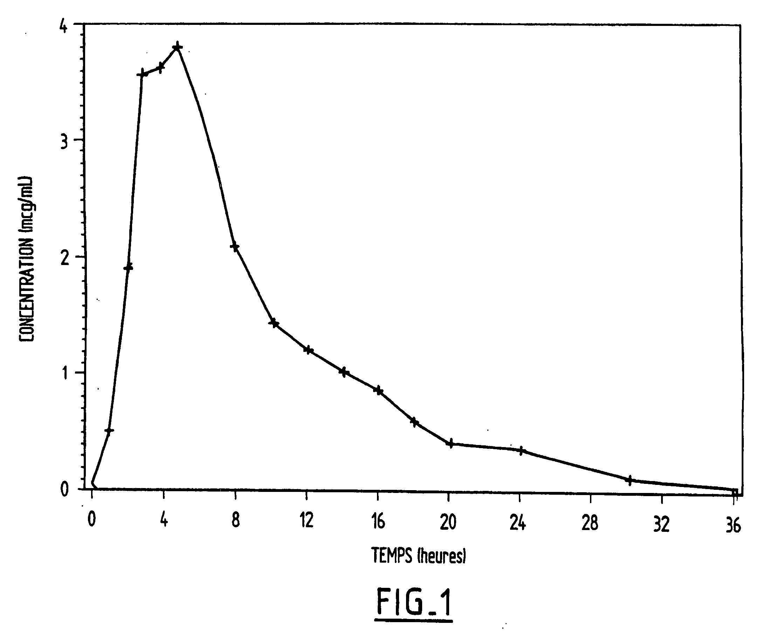

Les figures 1 à 3 donnent les résultats de dissolution in vivo respectivement des gélules des exemples 1 à 3. La courbe représente l'évolution au cours du temps de la concentration plasmatique moyenne en kétoprofène, mesurée sur cinq patients, après administration à chacun d'eux d'une gélule taille 1 contenant une quantité de microgranules de l'exemple 1 correspondant à 200 mg de kétoprofène.

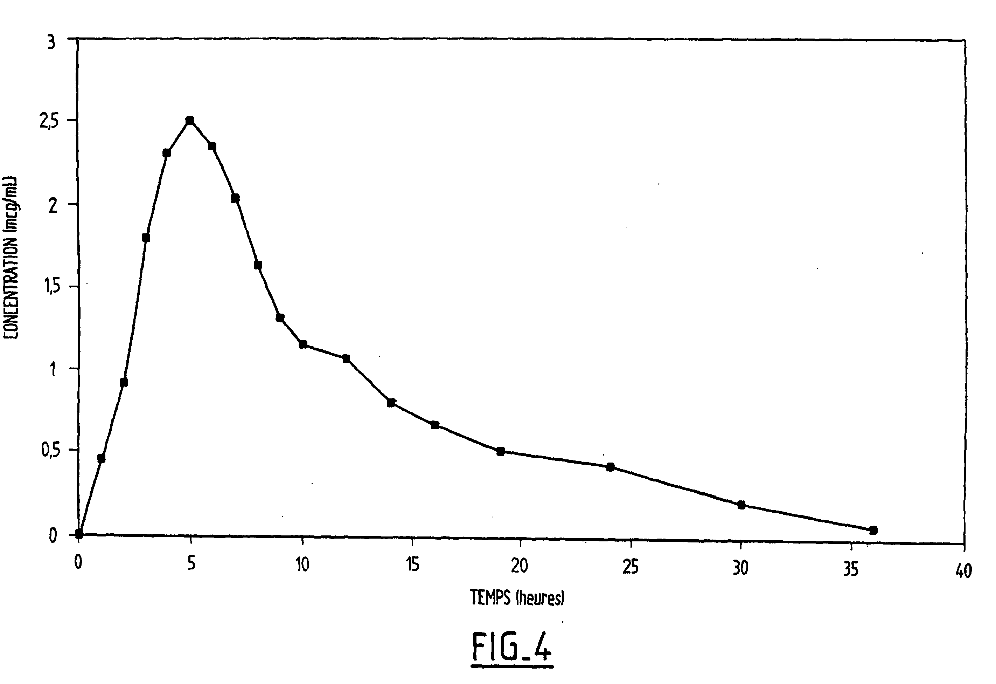

La figure 4 donne les résultats de dissolution in vivo de gélules de l'exemple 4. La courbe représente l'évolution au cours du temps de la concentration plasmatique mesurée sur 20 patients, après administration à chacun d'eux d'une gélule taille 1 contenant une quantité de microgranules de l'exemple correspondant à 200 mg de kétoprofène.

Les exemples suivants illustrent l'invention sans en limiter la portée.

### EXEMPLE 1 (microgranules YXK 002 A ; gélules 8308).

### a) Préparation des microgranules.

On prépare des microgranules de la composition suivante par le procédé décrit ci-après,

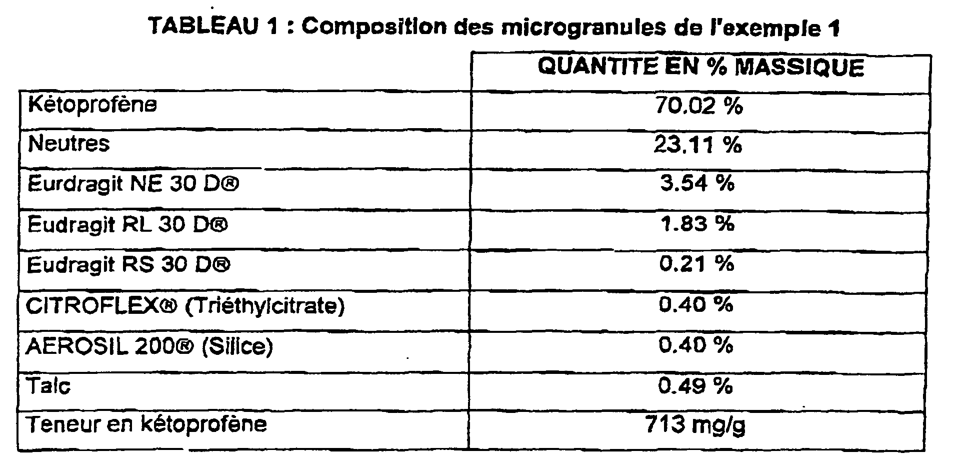

### . Préparation de la suspension de montage

La suspension est préparée dans un récipient inox. L'eau purifiée est versée dans le récipient puis mise en agitation. 15 % en poids d'Eudragit NE 30D® sont lentement incorporés à l'eau après agitation vigoureuse du bidon le contenant.

L'agitation est maintenue jusqu'à homogénéité de la suspension.

### . Montage du kétoprofène sur les grains supports neutres

Des grains supports Neutres 30® sont placés dans une turbine à dragéification en rotation.

Le montage du principe actif, est effectué sur les Neutres 30®, par pulvérisation discontinue de la suspension de montage contenant le liant en alternance avec des séquences de poudrage du kétoprofène, et des séquences de repos.

La masse de microgranules obtenue est tamisée sur une grille d'ouverture de maille allant de 0,355 à 1,25 mm. Les microgranules sont ensuite séchés dans la turbine en rotation à 60°C et ce, pendant 10 heures.

### . Préparation de la suspension d'enrobage

On prépare une suspension aqueuse à 15 % contenant les excipients suivants :

| | |
|---|---|
| EUDRAGIT RL 30D® | 90,0 % de la masse totale de l'extrait sec Eudragit |
| EUDRAGIT RS 30D® | 10,0 % de la masse totale de l'extrait sec Eudragit |
| SILICE | 20,0 % de la masse totale de l'extrait sec Eudragit |
| TRIETHYL CITRATE | 20,0 % de la masse totale de l'extrait sec Eudragit |

La suspension est préparée dans un récipient inox dans lequel on introduit l'eau purifiée et L'Eudragit RS-30D® après agitation vigoureuse du bidon le contenant. L'agitation est maintenue jusqu'à homogénéité de la suspension. L'Eudragit RL-30D® est ensuite incorporé après agitation vigoureuse du bidon le contenant.

La silice puis le triéthyl-citrate sont incorporés par petites quantités à la suspension. L'agitation est maintenue jusqu'à homogénéité du mélange, puis durant tout l'enrobage.

### . Enrobage des microgranules de kétoprofène

Les microgranules à enrober sont placés dans une turbine à dragéification. L'enrobage des microgranules est effectué par pulvérisation continue de la suspension décrite ci-dessus.

La masse de microgranules obtenue est tamisée sur une grille d'ouverture de maille comprise entre 0,355 et 1,25 mm.

Les microgranules sont ensuite séchés en turbine en rotation à une température de 35°C pendant 7 heures.

L'étape d'enrobage est effectuée selon ce protocole, en une ou plusieurs phases successives si nécessaire, afin d'obtenir la cinétique désirée.

Les microgranules sont lubrifiés avec du talc pendant une minute.

La teneur en kétoprofène est déterminée en dissolvant les microgranules dans un mélange méthanol/eau (3/1 en volume), puis en mesurant l'absorbance UV à 257 nm.

### b) Profil de dissolution in vitro des microgranules.

Le profil de dissolution in vitro des microgranules obtenus précédemment est établi en suivant le protocole suivant :

La quantité des microgranules correspondant à 200 mg de kétoprofène, tel qu'obtenus précédemment sont dissous dans 900 ml d'eau à pH = 6,9, à 37°C, sous agitation à 75 rpm, pendant 24 heures, dans un appareil à palettes décrit dans la 3^{ème} édition de la Pharmacopée européenne.

La concentration en kétoprofène est mesurée par spectrophotométrie UV à 260 nm.

Les résultats sont présentés dans le tableau 2 ci-après

**TABLEAU 2 :**

| **Dissolution in vitro des microgranules de l'exemple 1** | |
|---|---|
| **TEMPS (heure)** | **Pourcentage de kétoprofène dissous** |
| 1 heure | 19.7 % |
| 2 heures | 34.8 % |
| 3 heures | 46.6 % |
| 4 heures | 55.2 % |
| 5 heures | 61.7 % |
| 6 heures | 66.6 % |
| 7 heures | 70.5 % |
| 8 heures | 73.7 % |

### c) Profil de dissolution in vitro de gélules 200 mg.

La masse de microgranules nécessaire pour obtenir l'équivalent de 200 mg de kétoprofène est calculée, puis mise en gélule de taille 1. Le profil de dissolution des gélules est élaboré dans les mêmes conditions que pour les microgranules.

Les résultats sont présentés dans le tableau suivant.

**TABLEAU 3 :**

| **Dissolution in vitro des gélules 200 mg contenant les microgranules de l'exemple 1** | |
|---|---|
| **TEMPS (heure)** | **Pourcentage de kétoprofène dissous** |
| 1 heure | 17.5 % |
| 2 heures | 31.7 % |
| 3 heures | 43.2 % |
| 4 heures | 52.0 % |
| 5 heures | 58.7 % |
| 6 heures | 63.9 % |
| 7 heures | 68.1 % |
| 8 heures | 71.6 % |
| 9 heures | 74.6 % |
| 10 heures | 77.2 % |
| 11 heures | 79.5 % |
| 12 heures | 81.3 % |

### d) Dissolution in vivo des gélules 200 mg.

Une étude in vivo est réalisée de façon randomisée, en double aveugle, two-way crossover, sur 5 personnes auxquelles on administre 1 seule gélule contenant 200 mg de kétoprofène.

Le tableau 4 et la figure 1 résument les résultats obtenus.

**TABLEAU 4 :**

| **Paramètres cinétiques in vivo des gélules 200 mg contenant les microgranules de l'exemple 1**. | |
|---|---|
| **Paramètre** | **Moyenne** |
| AUC ₀₋ₜ (µg.h.ml⁻¹) | 35,8 |
| AUC_{inf} (µg.h.ml⁻¹) | 39,0 |
| Cₘₐₓ (µg.ml⁻¹) | 4,4 |
| Tₘₐₓ (h) • | 3,5 |
| T_{1/2} (h) | 7,7 |

### e) Etude de stabilité de gélules 100 mg

Les gélules 100 mg conditionnés dans des blister PVC/alu placées dans des conditions de stockage normales (25°C, 60 % d'humidité relative) font l'objet d'une étude de stabilité pendant 28 mois.

Les résultats de cette étude sont résumés dans le tableau 5 :

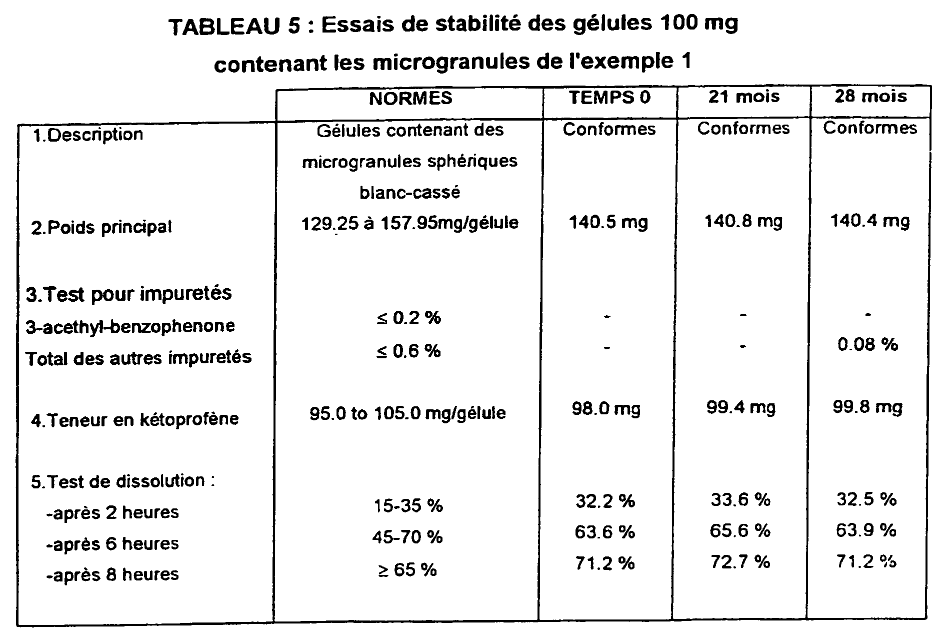

### EXEMPLE 2 (microgranules YXK 002 A1 ; gélules 8494)

### a) Préparation des microgranules

Les microgranules de la composition suivante :

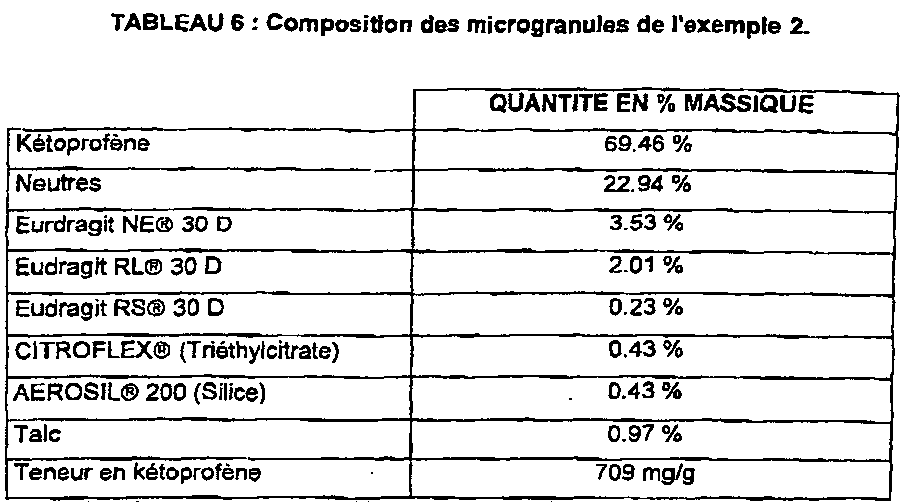

sont préparés selon le procédé décrit dans l'exemple 1.

### b) Profil de dissolution in vitro des microgranules.

Le profil de dissolution in vitro des microgranules, obtenu dans les mêmes conditions que l'exemple 1, est présenté dans le tableau 7.

**TABLEAU 7 :**

| **Dissolution in vitro des microgranules de l'exemple 2.** | |
|---|---|
| **TEMPS (heure)** | **Pourcentage de kétoprofène dissous** |
| 1 heure | 13.1 % |
| 2 heures | 24.1 % |
| 3 heures | 33.5 % |
| 4 heures | 41.2 % |
| 5 heures | 47.6 % |
| 6 heures | 52.9 % |
| 7 heures | 57.3 % |
| 8 heures | % |

### a) Profil de dissolution in vitro des gélules 200 mg :

Le profil de dissolution in vitro des gélules contenant une quantité de microgranules obtenus précédemment, correspondant à 200 mg de kétoprofène est déterminé dans les conditions de l'exemple 1.

Les résultats sont présentés dans le tableau 8.

**TABLEAU 8 :**

| **Dissolution in vitro des gélules 200 mg contenant les microgranules de l'exemple 2.** | |
|---|---|
| **TEMPS (heure)** | **Pourcentage de kétoprofène dissous** |
| 1 heure | 14.3 % |
| 2 heures | 26.5 % |
| 3 heures | 36.8 % |
| 4 heures | 45.2 % |
| 5 heures | 51.9 % |
| 6 heures | 57.2 % |
| 7 heures | 61.5 % |
| 8 heures | 65.1 % |
| 9 heures | 68.3 % |
| 10 heures | 71.1 % |
| 11 heures | 73.5 % |
| 12 heures | 75.5 % |

### d) Profil de dissolution in vivo des gélules 200 mg.

Une étude in vivo est réalisée de façon randomisée, "three-way cross over ?", sur 5 personnes auxquelles on administre une seule gélule contenant 200 mg de kétoprofène.

Le tableau 9 et la figure 2 résument les résultats obtenus.

**TABLEAU 9 :**

| **Paramètres cinétiques in vivo des gélules 200 mg contenant les microgranules de l'exemple 2.** | |
|---|---|
| **Paramètre** | **Moyenne** |
| AUC ₀₋ₜ (µg.h/ml) | 27.87 |
| AUC_{inf} (µg.h/ml) | 31.88 |
| Cₘₐₓ (µg.ml⁻¹) | 3.16 |

### EXEMPLE 3 (microgranules YKET 003 ; gélules 8500)

### a) Préparation des microgranules :

Les microgranules de la composition suivante :

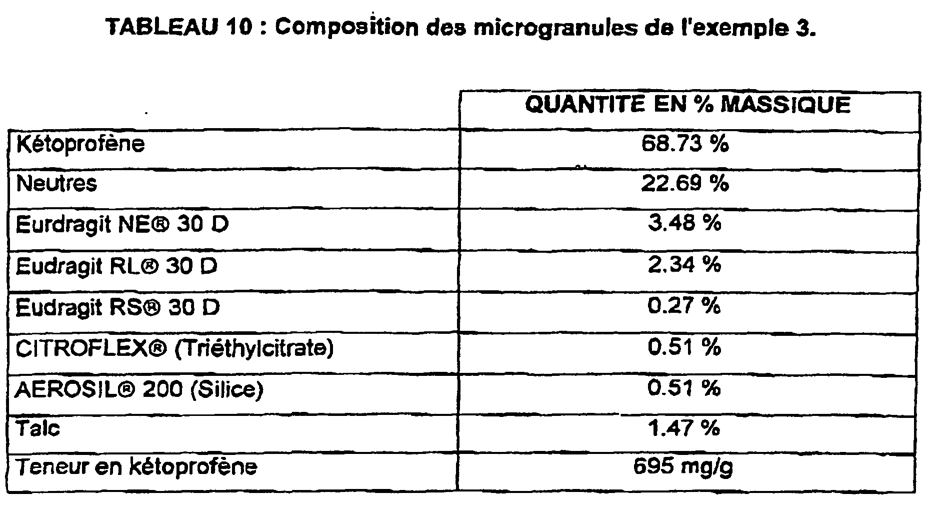

sont préparés selon le procédé décrit dans l'exemple 1.

### b) Profil de dissolution in vitro des microgranules :

Le profil de dissolution in vitro des microgranules et des gélules, obtenu dans les mêmes conditions que l'exemple 1 est présenté dans le tableau 11.

**TABLEAU 11 :**

| **Dissolution in vitro des microgranules de l'exemple 3.** | |
|---|---|
| **TEMPS (heure)** | **Pourcentage de kétoprofène dissous** |
| 1 heure | 12.1 % |
| 2 heures | 22.7 % |
| 3 heures | 32.1 % |
| 4 heures | 40.3 % |
| 5 heures | 47.4 % |
| 6 heures | 53.3 % |
| 7 heures | 58.1 % |
| 8 heures | 62.2 % |
| 9 heures | 65.7 % |
| 10 heures | 68.8 % |
| 11 heures | 71.5 % |
| 12 heures | 74.0 % |

### c) Profil de dissolution in vitro des gélules 200 mg.

Le profil de dissolution in vitro des gélules contenant une quantité de microgranules obtenus précédemment, correspondant à 200 mg de kétoprofène est déterminé dans les conditions de l'exemple 1.

Les résultats sont présentés dans le tableau 12.

**TABLEAU 12 :**

| **Dissolution in vitro des gélules 200 mg contenant les microgranules de l'exemple 3.** | |
|---|---|
| **TEMPS (heure)** | **Pourcentage de kétoprofène dissous** |
| 1 heure | 11.3 % |
| 2 heures | 21.2 % |
| 3 heures | 29.9 % |
| 4 heures | 37.8 % |
| 5 heures | 44.4 % |
| 6 heures | 49.9 % |
| 7 heures | 54.7 % |
| 8 heures | 58.7 % |
| 9 heures | 62.2 % |
| 10 heures | 65.3 % |
| 11 heures | 67.9 % |
| 12 heures | 70.3 % |

### d) Profil de dissolution in vivo des gélules 200 mg.

Une étude in vivo est réalisée de façon randomisée, three-way cross over, sur 5 personnes auxquelles on administre une seule gélule contenant 200 mg de kétoprofène.

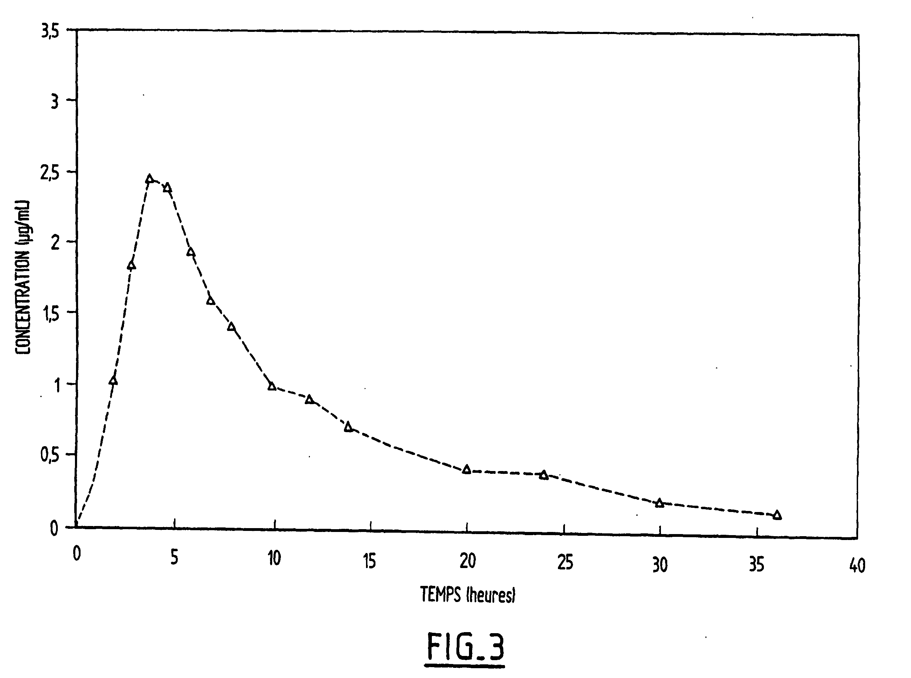

Le tableau 13 et la figure 3 résument les résultats obtenus

**TABLEAU 13 :**

| **Paramètres cinétiques in vivo des gélules 200 mg contenant les microgranules de l'exemple 3.** | |
|---|---|
| **Paramètre** | **Moyenne** |
| AUC ₀₋ₜ (µg.h/ml) | 25.75 |
| AUC_{inf} (µg.h/ml) | 30.39 |
| Cₘₐₓ (µg.ml⁻¹) | 2.47 |

### EXEMPLE 4 (microgranules YKET 006 ; gélules 8571).

### a) Préparation des microgranules.

Les microgranules de la composition suivante :

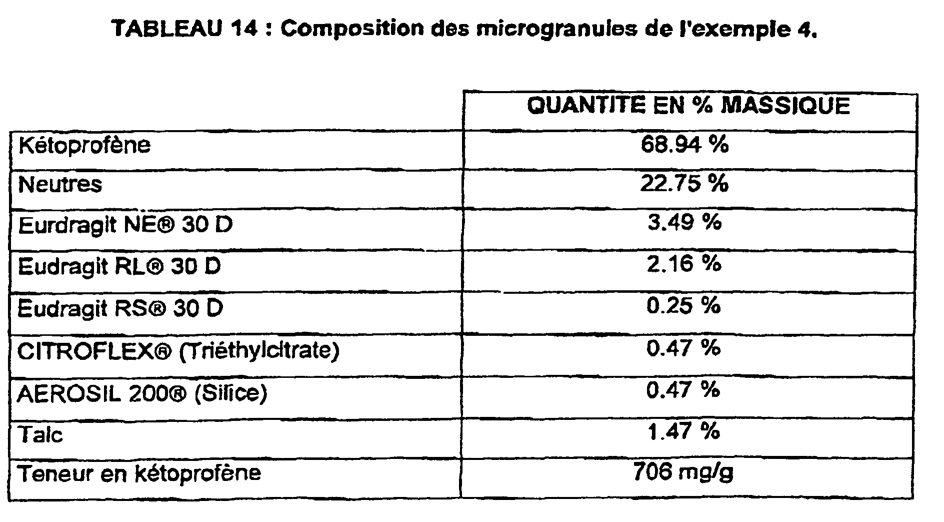

sont préparés selon le procédé décrit dans l'exemple 1.

### b) Profil de dissolution in vitro des microgranules et des gélules :

Le profil de dissolution in vitro des microgranules et des gélules, obtenu dans les mêmes conditions que l'exemple 1 est présenté dans le tableau 15.

**TABLEAU 15 :**

| **Dissolution in vitro des microgranules de l'exemple 4.** | |
|---|---|
| **TEMPS (heure)** | **Pourcentage de kétoprofène dissous** |
| 1 heure | 12.9 % |
| 2 heures | 24.3 % |
| 3 heures | 34.2 % |
| 4 heures | 42.7 % |
| 5 heures | 49.7 % |
| 6 heures | 55.6 % |
| 7 heures | 60.4 % |
| 8 heures | 64.5 % |
| 9 heures | 67.9 % |
| 10 heures | 70.9 % |
| 11 heures | 73.4 % |
| 12 heures | 75.7 % |

### c) Profil de dissolution in vitro des gélules 200 mg :

Le profil de dissolution in vitro des gélules contenant une quantité de microgranules obtenus précédemment, correspondant à 200 mg de kétoprofène est déterminé dans les conditions de l'exemple 1.

Les résultats sont présentés dans le tableau 16.

**TABLEAU 16 :**

| **Dissolution in vitro des gélules 20 mg contenant les microgranules de l'exemple 4** | |
|---|---|
| **TEMPS (heure)** | **Pourcentage de kétoprofène dissous** |
| Test de dissolution | |
| 1 heure | 11.9 % |
| 2 heures | 22.1 % |
| 3 heures | 31.2 % |
| 4 heures | 39.2 % |
| 5 heures | 46.1 % |
| 6 heures | 51.8 % |
| 7 heures | 56.8 % |
| 8 heures | 61.1 % |
| 9 heures | 64.5 % |
| 10 heures | 68.0 % |
| 11 heures | 70.8 % |
| 12 heures | 73.3 % |

### d) Profil de dissolution in vivo des gélules 20 mg.

Une étude in vivo est réalisable de façon randomisée, two-way cross over, sur 20 personnes auxquelles on administre une seule gélule contenant 20 mg de kétoprofène.

Le tableau 17 et la figure 4 résument les résultats obtenus.

**TABLEAU 17 :**

| **Paramètres cinétiques in vivo des gélules 200 mg contenant les microgranules de l'exemple 4.** | |
|---|---|
| **Paramètre** | **Moyenne** |
| AUC ₀₋ₜ (µg.h/ml) | 28.01 |
| AUC_{inf} (µg.h/ml) | 29.94 |
| Cₘₐₓ (µg.ml⁻¹) | 2.92 |

### e) Etude de stabilité des gélules de 200 mg.

Des gélules 200 mg conditionnés dans des blister PVC/Alu font l'objet d'une étude de stabilité pendant 16 mois dans des conditions normales de stockage (21°C, 60 % d'humidité relative). Les résultats sont présentés dans le tableau ci-dessous.

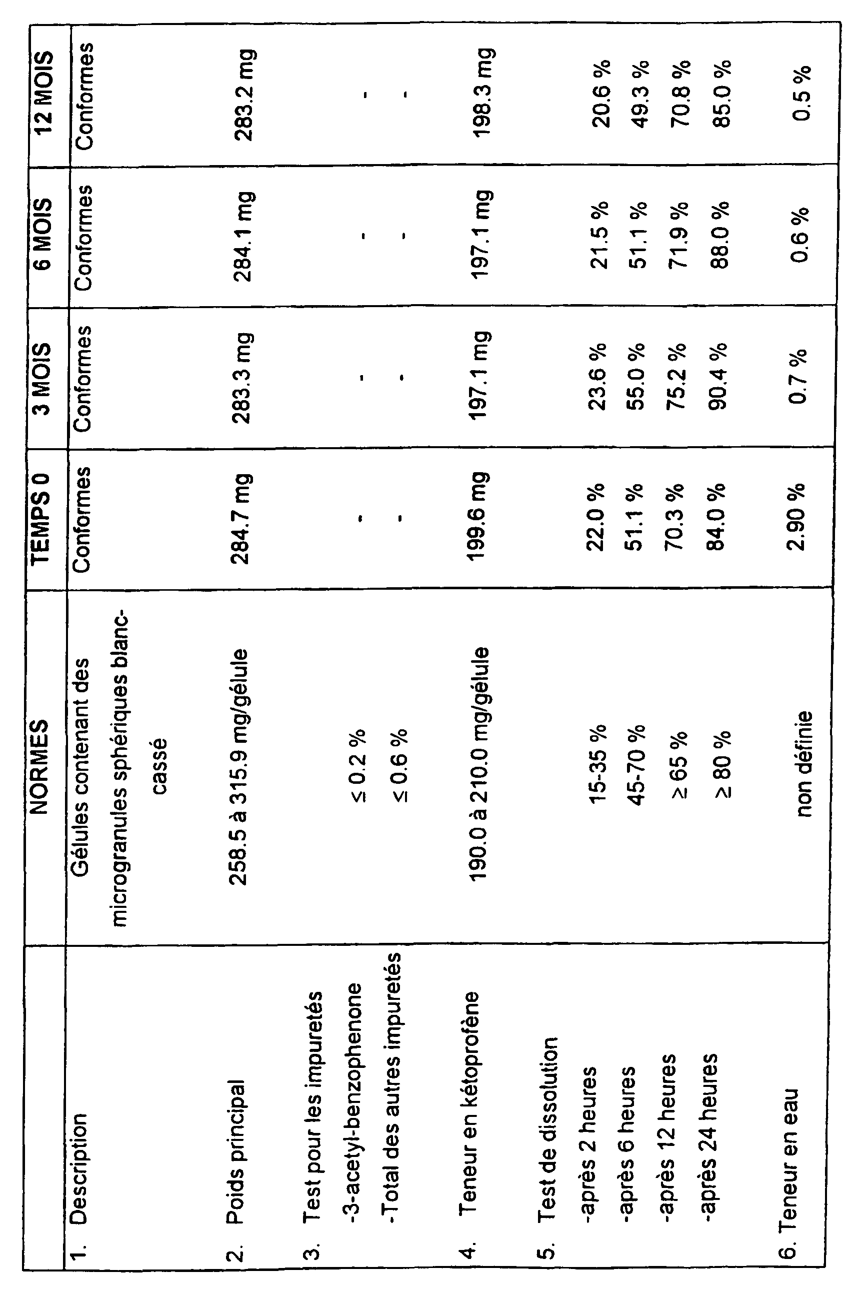

## Revendications

1. Microgranules de kétoprofène à libération prolongée, chacun constitués d'un noyau actif contenant du kétoprofène enrobé d'une couche polymérique permettant la libération prolongée du principe actif **caractérisés en ce que** la couche polymérique contient une dispersion aqueuse à 30 % du copolymère poly(éthyl acrylate, méthyl méthacrylate, chlorure de triméthylammonioéthyl méthacrylate) dans la proportion 1/2/0,2, et une dispersion aqueuse à 30 % du copolymère poly(éthyl acrylate, méthyl méthacrylate, chlorure de triméthylammonioéthyl méthacrylate) dans la proportion 1/2/0,1, dans une proportion massique comprise entre 80/20 et 95/5, de préférence sensiblement égale à 90/10.

2. Microgranules selon la revendication 1, **caractérisés en ce que** la masse totale de dispersion aqueuse à 30 % du copolymère poly(éthyl acrylate, méthyl méthacrylate, chlorure de triméthylammonioéthyl méthacrylate) dans la proportion 1/2/0,2, et de dispersion aqueuse à 30 % du copolymère poly(éthyl acrylate, méthyl méthacrylate, chlorure de triméthylammonioéthyl méthacrylate) dans la proportion 1/2/0,1 représente entre 1 à 8 % de la masse du microgranule, de préférence 2 et 5%.

3. Microgranules selon la revendication 1 ou 2, **caractérisés en ce que** la couche entérique enrobant le noyau contient un agent plastifiant, comme le triéthyl-citrate, et/ou un agent lubrifiant, comme la silice.

4. Microgranules selon l'une des revendications précédentes, **caractérisés en ce que** le kétoprofène représente 50 à 80 % en poids du microgranule, de préférence 65 à 75 %.

5. Microgranules selon la revendication 1, **caractérisés en ce que** le noyau est constitué d'un grain support neutre enrobé de kétoprofène et d'un agent liant, par exemple un polyacrylate.

6. Microgranules selon la revendication 5, **caractérisés en ce que** le grain support neutre représente 10 à 40 % en poids de la masse du microgranule, de préférence 20 à 25 %.

7. Procédé de préparation des microgranules selon l'une des revendications 1 à 6, **caractérisé en ce qu'**il comprend une étape d'enrobage du noyau actif par pulvérisation d'une solution aqueuse contenant une dispersion aqueuse à 30 % du copolymère poly(éthyl acrylate, méthyl méthacrylate, chlorure de triméthylammonioéthyl méthacrylate) dans la proportion 1/2/0,2, et une dispersion aqueuse à 30 % du copolymère poly(éthyl acrylate, méthyl méthacrylate, chlorure de triméthylammonioéthyl méthacrylate) dans la proportion 1/2/0,1.

8. Procédé selon la revendication 7, **caractérisé en ce que** le noyau actif est obtenu par montage du kétoprofène sur des grains supports neutres avec une solution aqueuse d'un agent liant comme un polyacrylate.

9. Composition pharmaceutique, **caractérisée en ce qu'**elle contient une masse de microgranules selon l'une des revendications 1 à 6 ou préparés selon le procédé de la revendication 7 ou 8, qui correspond à une dose journalière de 50 à 300 mg environ de kétoprofène, de préférence 100 mg à 200 mg.

## Patentansprüche

1. Mikrogranalien von Ketoprofen mit verzögerter Freisetzung, jede bestehend aus einem Ketoprofen enthaltenden aktiven Kern, der mit einer polymeren Schicht umhüllt ist, welche die verzögerte Freisetzung des Wirkstoffs ermöglicht, **dadurch gekennzeichnet, dass** die polymere Schicht eine 30%-ige wässrige Dispersion des Copolymers Poly(ethylacrylat, methylmethacrylat, trimethylammonioethylmethacrylatchlorid) im Verhältnis 1/2/0,2 und eine 30%-ige wässrige Dispersion des Copolymers Poly(ethylacrylat, methylmethacrylat, trimethylammonioethylmethacrylatchlorid) im Verhältnis 1/2/0,1 in einem Gewichtsverhältnis zwischen 80/20 und 95/5 einschließlich, vorzugsweise etwa gleich 90/10, enthält.

2. Mikrogranalien nach Anspruch 1, **dadurch gekennzeichnet, dass** das Gesamtgewicht der 30%-igen wässrigen Dispersion des Copolymers Poly(ethylacrylat, methylmethacrylat, trimethylammonioethylmethacrylatchlorid) im Verhältnis 1/2/0,2 und der 30%-igen wässrigen Dispersion des Copolymers Poly(ethylacrylat, methylmethacrylat, trimethylammonioethylmethacrylatchlorid) im Verhältnis 1/2/0,1 zwischen 1 bis 8 % des Gewichts der Mikrogranalie, vorzugsweise zwischen 2 und 5 % darstellt.

3. Mikrogranalien nach Anspruch 1 oder 2, **dadurch gekennzeichnet, dass** die enterische Schicht, welche den Kern umhüllt, ein weichmachendes Mittel, wie Triethylcitrat, und/oder ein Gleitmittel, wie Siliciumdioxid, enthält.

4. Mikrogranalien nach einem der vorangehenden Ansprüche, **dadurch gekennzeichnet, dass** das Ketoprofen 50 bis 80 Gewichts-% der Mikrogranalie, vorzugsweise 65 bis 75 % darstellt.

5. Mikrogranalien nach Anspruch 1, **dadurch gekennzeichnet, dass** der Kern aus einem neutralen Trägerkorn besteht, das von Ketoprofen und einem Bindemittel, zum Beispiel einem Polyacrylat, umhüllt ist.

6. Mikrogranalien nach Anspruch 5, **dadurch gekennzeichnet, dass** das neutrale Trägerkorn 10 bis 40 Gewichts-% des Gewichts der Mikrogranalie, vorzugsweise 20 bis 25 % darstellt.

7. Verfahren zur Herstellung von Mikrogranalien nach einem der Ansprüche 1 bis 6, **dadurch gekennzeichnet, dass** es einen Schritt der Umhüllung des aktiven Kerns durch Zerstäuben einer wässrigen Lösung umfasst, die eine 30%-ige wässrige Dispersion des Copolymers Poly(ethylacrylat, methylmethacrylat, trimethylammonioethylmethacrylatchlorid) im Verhältnis 1/2/0,2 und eine 30%-ige wässrige Dispersion des Copolymers Poly(ethylacrylat, methylmethacrylat, trimethylammonioethylmethacrylatchlorid) im Verhältnis 1/2/0,1 enthält.

8. Verfahren nach Anspruch 7, **dadurch gekennzeichnet, dass** der aktive Kern durch Aufbringen des Ketoprofens auf neutrale Trägerkörner mit einer wässrigen Lösung eines Bindemittels, wie eines Polyacrylats, erhalten wird.

9. Pharmazeutische Zusammensetzung, **dadurch gekennzeichnet, dass** sie eine Masse von Mikrogranalien gemäß einem der Ansprüche 1 bis 6 oder hergestellt gemäß dem Verfahren nach Anspruch 7 oder 8 enthält, die einer täglichen Dosis von etwa 50 bis 300 mg Ketoprofen, vorzugsweise 100 mg bis 200 mg, entspricht.

## Claims

1. Sustained-release ketoprofen microgranules, each consisting of an active core containing ketoprofen coated with a polymeric layer which allows sustained release of the active principle, **characterized in that** the polymeric layer contains a 30% aqueous dispersion of the copolymer poly(ethyl acrylate, methyl methacrylate, trimethylammonioethyl methacrylate chloride) in the proportion 1/2/0.2 and a 30% aqueous dispersion of the copolymer poly(ethyl acrylate, methyl methacrylate, trimethylammonioethyl methacrylate chloride) in the proportion 1/2/0.1, in a mass proportion of between 80/20 and 95/5, preferably approximately equal to 90/10.

2. Microgranules according to Claim 1, **characterized in that** the total mass of 30% aqueous dispersion of the copolymer poly(ethyl acrylate, methyl methacrylate, trimethylammonioethyl methacrylate chloride) in the proportion 1/2/0.2 and of 30% aqueous dispersion of the copolymer poly(ethyl acrylate, methyl methacrylate, trimethylammonioethyl methacrylate chloride) in the proportion 1/2/0.1, represents between 1 and 8% of the mass of the microgranule, preferably 2 and 5%.

3. Microgranules according to Claim 1 or 2, **characterized in that** the enteric layer coating the core contains a plasticizer, such as triethyl citrate, and/or a lubricant, such as silica.

4. Microgranules according to one of the preceding claims, **characterized in that** the ketoprofen represents 50 to 80% by weight of the microgranule, preferably 65 to 75%.

5. Microgranules according to Claim 1, **characterized in that** the core consists of a neutral support grain coated with ketoprofen and a binding agent, for example a polyacrylate.

6. Microgranules according to Claim 5, **characterized in that** the neutral support grain represents 10 to 40% by weight of the mass of the microgranule, preferably 20 to 25%.

7. Method for preparing the microgranules according to one of Claims 1 to 6, **characterized in that** it comprises a step for coating the active core by spraying an aqueous solution containing a 30% aqueous dispersion of the copolymer poly(ethyl acrylate, methyl methacrylate, trimethylammonioethyl methacrylate chloride) in the proportion 1/2/0.2 and a 30% aqueous dispersion of the copolymer poly(ethyl acrylate, methyl methacrylate, trimethylammonioethyl methacrylate chloride) in the proportion 1/2/0.1.

8. Method according to Claim 7, **characterized in that** the active core is obtained by assembling the ketoprofen on neutral support grains with an aqueous suspension of a binding agent such as a polyacrylate.

9. Pharmaceutical composition, **characterized in that** it contains a mass of microgranules according to one of Claims 1 to 6 or prepared according to the method of Claim 7 or 8, which corresponds to a daily dose of 50 to 300 mg approximately of ketoprofen, preferably 100 mg to 200 mg.
